# EUROPEAN PATENT SPECIFICATION

(11) **EP 3 639 819 B1**
(45) Date of publication and mention of the grant of the patent: **10.11.2021**
(21) Application number: 18818773.6
(22) Date of filing: 15.06.2018
(51) Int. Cl.: A61K 9/00, A61K 47/14, A61K 31/167, A61K 31/196

(54) **ANTI-INFLAMMATORY AND ANALGESIC DRUG FOR EXTERNAL USE**
ENTZÜNDUNGSHEMMENDES UND SCHMERZSTILLENDES MEDIKAMENT ZUR ÄUSSERLICHEN ANWENDUNG
PRÉPARATION ANALGÉSIQUE ET ANTI-INFLAMMATOIRE À USAGE EXTERNE

(30) Priority: 16.06.2017 JP 2017119144
(43) Date of publication of application: 22.04.2020
(73) Proprietor: Medrx Co., Ltd., Kagawa 769-2712 (JP)
(72) Inventor: ISHIBASHI, Masaki, Higashikagawa-shi Kagawa 769-2712 (JP); HAMAMOTO, Hidetoshi, Higashikagawa-shi Kagawa 769-2712 (JP)
(74) Representative: Vossius & Partner Patentanwälte Rechtsanwälte mbB
(86) International application number: PCT/JP2018/022847
(87) International publication number: WO 2018/230687

(56) References cited:
- EP-A1- 2 210 599
- WO-A1-2009/060629
- WO-A1-2014/157436
- WO-A2-2010/087947
- JP-A- 2006 328 059
- JP-A- 2007 022 942
- JP-A- 2007 153 849
- US-A1- 2004 068 007

## Description

### TECHNICAL FIELD

This patent application claims the benefit of Japanese Patent Application No. 2017-119144.

The present disclosure relates to an external preparation comprising an equimolar salt of lidocaine and lactic acid (lactic acid salt of lidocaine) and diclofenac or a salt thereof.

### BACKGROUND ART

Various external preparations comprising a non-steroidal antiphlogistic analgesic and a local anesthetic are suggested (e.g., Patent Documents 1-5). One of the commonly-used non-steroidal antiphlogistic analgesics, diclofenac sodium had low solubility in solvents, and thus was difficult to be prepared as an external preparation such as patch preparation. Even if such external preparation was prepared, the medicinal effect of diclofenac was not sometimes produced because of the insufficient skin permeability. In order to enhance the skin permeability of diclofenac, some attempts to form an ionic liquid by combining diclofenac with a local anesthetic have been done (Patent Documents 2-5). As a result, it has been reported that certain results such as the depression of melting point due to ion pair formation, the improvement of solubility in organic solvents, and the reduction of skin irritation were achieved. However, the solubility of diclofenac-lidocaine salt in solvents was still insufficient. Hence, further improvement of the preparations has been desired. It has not been reported that external preparations comprising diclofenac and lidocaine could be clinically used.

As techniques for easily dissolving lidocaine in an organic solvent to enhance the transdermal absorbability of lidocaine, it has been known that lidocaine is reacted with an equimolar amount of lactic acid to provide lactic acid salt of lidocaine in the ionic liquid form (Patent Document 6).

### PRIOR ART DOCUMENTS

### PATENT DOCUMENTS

Patent Document 1: JP 2002-238699
Patent Document 2: JP 2003-335663
Patent Document 3: JP 2004-323502
Patent Document 4: JP 2005-145931
Patent Document 5: JP 2005-82512
Patent Document 6: WO 2009/060629

### SUMMARY OF INVENTION

### (PROBLEM TO BE SOLVED BY THE INVENTION)

An object of the present disclosure is to provide an external preparation comprising lidocaine and diclofenac or a salt thereof as active ingredients which exhibits the improved transdermal absorbability of both ingredients and the skin permeability suitable for clinical use.

### (MEANS FOR SOLVING THE PROBLEMS)

The present inventors have extensively studied to reach the above object, and then have found that when an appropriate amount of diclofenac sodium is dissolved in an equimolar salt of lidocaine and lactic acid (ionic liquid), lidocaine and diclofenac are kept in the solution state without the precipitation of crystals thereof, and thus the skin permeability suitable for clinical use can be achieved. In addition, the present inventors have found that lidocaine and diclofenac or a salt thereof in an external preparation are contained in the state uniformly compatibilized with or dispersed in the adhesive layer of the preparation without the precipitation of crystals thereof even when the external preparation comprising high concentrations of lidocaine and diclofenac or a salt thereof as active ingredients is prepared, and thus the skin permeability of both ingredients can be enhanced. Based upon the new findings, the present invention has been completed.

Specifically, the present disclosure provides the following embodiments.
[1] An external preparation comprising lactic acid salt of lidocaine and diclofenac or a salt thereof, wherein lactic acid salt of lidocaine is contained in an amount of 2-5 moles per mole of diclofenac or a salt thereof.
[2] The external preparation of the item [1], wherein the amount of lactic acid salt of lidocaine is 5-40% by weight.
[3] The external preparation of the item [1] or [2], wherein the amount of diclofenac or a salt thereof is 1-20% by weight.
[4] The external preparation of any of the items [1]-[3], further comprising an ester.
[5] The external preparation of the item [4], wherein the ester is diethyl sebacate, methyl laurate, diisopropyl adipate, isopropyl myristate, propylene carbonate, or a mixture thereof.
[6] The external preparation of any of the items [1]-[5], which is matrix-type patch preparation (tape preparation).
[7] The external preparation of the item [6], wherein an adhesive layer therein comprises a polymer with a dispersed solution comprising lactic acid salt of lidocaine and diclofenac or a salt thereof.
[8] A process for preparing the external preparation of the item [1], comprising:
   mixing lidocaine and lactic acid to provide lactic acid salt of lidocaine which is liquid at ambient temperature; and
   dissolving diclofenac or a salt thereof into lactic acid salt of lidocaine.

### (EFFECTS OF THE INVENTION)

According to the present disclosure, two types of analgesics with different action mechanisms (lidocaine and diclofenac) can sufficiently permeate the skin. Hence, an analgesic that is extremely excellent in reducing both inflammatory pain and neuropathic pain is provided. Also, both lidocaine and diclofenac are in a solution state. Hence, when the drugs are prepared as a tape preparation, the reduction of the adhesive force of the preparation to the skin can be prevented.

### BRIEF DESCRIPTION OF THE DRAWINGS

Fig.1 shows a phase diagram for three component mixtures (ternary phase diagram) representing the state in which lidocaine, lactic acid and diclofenac sodium are mixed in relative proportions. • shows the clear solution state without the precipitation of crystals of lidocaine and diclofenac, ■ shows the cloudy solution state without the precipitation of crystals of lidocaine and diclofenac, and ▲ shows the state in which some or all of lidocaine and/or diclofenac sodium are not dissolved and solids thereof remain.
Fig.2 shows a graph showing the changes in plasma diclofenac concentration in the pharmacokinetic study using miniature pig.
Fig.3 shows a graph showing the changes in plasma lidocaine concentration in the pharmacokinetic study using miniature pig.

### DESCRIPTION OF EMBODIMENTS

### [Lactic acid salt of lidocaine (Ionic liquid)]

Lactic acid salt of lidocaine is an ionic liquid (an ambient temperature molten salt) produced by the reaction of lidocaine with an equimolar amount of lactic acid. Lactic acid salt of lidocaine is produced by mixing lidocaine and an equimolar amount of lactic acid in the presence or absence of solvent and heating the mixture (for example, at 80°C). Also, lactic acid salt of lidocaine can be produced by mixing the ingredients at room temperature. Lidocaine and lactic acid may be partially reacted to form an equimolar salt of lidocaine and lactic acid, and there may be unreacted lidocaine and/or lactic acid. Lidocaine is in the solid form at ambient temperature, whereas lactic acid salt of lidocaine is in the viscous liquid form at ambient temperature.

The amount of lactic acid salt of lidocaine is, for example, 5-40% by weight, preferably 10-35% by weight, more preferably 20-30% by weight, and most preferably 25-30% by weight. The amount of lactic acid salt of lidocaine may be about 5% by weight, about 10% by weight, about 15% by weight, about 20% by weight, about 25% by weight, about 30% by weight, about 35% by weight or about 40% by weight.

### [Diclofenac or salt thereof]

Diclofenac or a salt thereof is basically used as a metal salt such as sodium salt and potassium salt, but is not limited thereto. The salt of diclofenac comprises a pharmaceutically acceptable salt such as a salt with free acid or organic base. In certain embodiments, diclofenac or a salt thereof is selected from diclofenac sodium or diclofenac potassium.

The external preparation of the present disclosure comprises lactic acid salt of lidocaine in an amount of 2-5 moles, 2-4 moles, or 2.5-3.5 moles per mole of diclofenac. When the mole ratio of lactic acid salt of lidocaine and diclofenac falls within the above range, the transdermal absorbability of both lidocaine and diclofenac is improved.

The amount of diclofenac or a salt thereof is, for example, 1-20% by weight, preferably 2-20% by weight, more preferably 5-10% by weight. For example, the amount of diclofenac sodium may be about 1% by weight, about 2% by weight, about 5% by weight, about 10% by weight, about 15% by weight, or about 20% by weight.

The external preparation of the present disclosure may comprise unreacted lidocaine and/or lactic acid.

In certain embodiments, diclofenac is dissolved in lactic acid salt of lidocaine. In such case, the external preparation of the present disclosure is neither a simple mixture of lidocaine, lactic acid, and diclofenac nor a mixture of lidocaine-diclofenac salt and lactic acid.

The external preparation of the present disclosure can be prepared by, for example, mixing lidocaine, lactic acid, and diclofenac, if necessary, mixing them with heating (e.g., at about 80°C). In certain embodiments, the external preparation of the present disclosure can be prepared by mixing lidocaine and lactic acid, if necessary, mixing them with heating to provide lactic acid salt of lidocaine and mixing diclofenac with lactic acid salt of lidocaine.

The external preparation of the present disclosure may further comprise an organic solvent with the effect for enhancing transdermal absorbability such as an alcohol, an ester, a fatty acid, and an amine. Two or more of the organic solvents may be used in combination. The amount of the organic solvent is, for example, 1-30% by weight, preferably 1-20% by weight, more preferably 1-10% by weight. Also, the organic solvent may comprise water in an amount of less than 1.0% by weight. In the external preparation of the present disclosure which is a matrix-type patch preparation (tape preparation), when the organic solvent used is excessively added, the adhesive layer in the patch preparation may be soften. As a result, it is sometimes difficult to prepare such preparation.

In certain embodiments, the alcohol may be monovalent alcohol such as lauryl alcohol, myristyl alcohol, oleyl alcohol, isostearyl alcohol, and cetyl alcohol; divalent alcohol such as propylene glycol, butylene glycol, dipropylene glycol, diisobutylene glycol, polyethylene glycol, and hexylene glycol; trivalent alcohol such as glycerin and hexanetriol. Also, the alcohol may be used alone or two or more of the alcohols may be used in combination.

In certain embodiments, the ester may be diethyl sebacate, methyl laurate, diisopropyl adipate, isopropyl myristate, or propylene carbonate. Also, the ester may be used alone or two or more of the esters may be used in combination.

In certain embodiments, the external preparation of the present disclosure comprises lactic acid salt of lidocaine, diclofenac or a salt thereof and isopropyl myristate. Isopropyl myristate is useful for enhancing the skin permeability of both lidocaine and diclofenac.

In certain embodiments, the fatty acid may be saturated or unsaturated fatty acid such as levulinic acid, capric acid, lauric acid, myristic acid, palmitic acid, stearic acid, isostearic acid, and oleic acid. Also, the fatty acid may be used alone or two or more of the fatty acids may be used in combination.

In certain embodiments, the amine may be monoethanolamine, diethanolamine, diisopropanolamine, triethanolamine, triisopropanolamine, ethylenediamine, and trishydroxymethylaminomethane. Also, the amine may be used alone or two or more of the amines may be used in combination.

The external preparation of the present disclosure may further comprise a surfactant. Examples of the surfactant include non-ionic surfactant such as monoglyceride stearate and polyoxyethylene castor oil; anionic surfactant such as sodium lauryl sulfate and potassium lauryl sulfate; and cationic surfactant such as benzalkonium chloride and stearyltrimethylammonium chloride. Two or more of the surfactants may be used in combination. The amount of the surfactant is, for example, 0.01-2% by weight, preferably 0.01-1% by weight.

In certain embodiments, the external preparation of the present disclosure may further comprise lidocaine or a salt thereof and/or lactic acid which do not form lactic acid salt of lidocaine.

### [Matrix-type patch preparation]

The external preparation of the present disclosure may comprise the structure with an adhesive layer comprising an active ingredient laminated on at least one side of a support. The external preparation of the present disclosure can be prepared as a matrix-type patch preparation by dispersing the solution comprising lactic acid salt of lidocaine and diclofenac or a salt thereof in an adhesive layer comprising an appropriate polymer (elastomer). The useful polymer may include an acrylic polymer, a rubber polymer, a silicone polymer, and a vinyl ether-based polymer. In certain embodiments, the rubber polymer such as styrene-isoprene-styrene block copolymer, styrene-butadiene-styrene block copolymer, polyisoprene, polyisobutylene, and polybutadiene can be preferably used. The amount of the rubber polymer may be about 5-40% by weight or about 5-20% by weight relative to the total weight of the dried adhesive layer. The amounts of the acrylic polymer and silicone polymer may be about 45-90% by weight or about 50-80% by weight relative to the total weight of the dried adhesive layer. Also, two or more of the polymers may be used in combination.

In certain embodiments, the adhesive layer may further comprise a filler such as hydrous silica, fumed silica, talc, crystalline cellulose, starch, carmellose, or metal salt of carmellose. The filler can improve not only the adhesion of the adhesive layer but also enhance the release rate of an active ingredient. The amount of the filler is, for example, the range of about 0.5-15% by weight, preferably about 1-10% by weight, more preferably about 2-5% by weight, relative to the total weight of the dried adhesive layer.

The adhesive layer may further comprise other additive(s) such as a tackifier, a softener, and an anti-oxidant. Examples of the tackifier include rosin ester, hydrogenated rosin ester, rosin maleate, alicyclic saturated hydrocarbon resin, terpene resin, and polyolefin resin. Examples of the softener include naphthenic processing oil; vegetable oil such as camellia oil and castor oil; liquid rubber such as liquid polybutene and liquid isoprene rubber; and liquid paraffin. Examples of the anti-oxidant include dibutyl hydroxy toluene, ascorbic acid, propyl gallate, sodium sulfite, and sodium pyrosulfite.

When the external preparation of the present disclosure is used as matrix-type patch preparation, the amount of lactic acid salt of lidocaine may be about 5-40%, about 10-35%, about 20-30%, or about 25-30% relative to the total weight of the dried adhesive layer.

As used herein, a numerical value accompanied with the term "about" is intended to include any value within the range of ± 2% of that value. The numerical range defined by both ends covers all values between the both ends as well as the values at the both ends. For example, "about 5%" means "5% ± 2%". However, the numerical value is never 0% or less.

The external preparation of the present disclosure may be used in the form of a dosage form such as cream, ointment, lotion, and cataplasm.

The amount of the external preparation of the present disclosure to be used varies depending on various factors such as the symptom and age of patients. The external preparation is preferably applied once to several times per day for adults. Furthermore preferably, the external preparation is applied once to twice per day. The frequency of administration may be increased depending on symptoms.

The external preparation of the present disclosure shows the following feature in the pharmacokinetic study using miniature pig. When the external preparation of the present disclosure is applied to the pig for 12 hours, the AUC₀₋₁₂ and Cmax values of diclofenac therein are about 6-7 times and about 8-9.5 times those of commercially-available diclofenac patch preparation comprising diclofenac in the same amount (Flector®), respectively. Also, the AUC₀₋₁₂ and Cmax values of lidocaine in the external preparation of the present disclosure applied for 12 hours are about 3.5-4.5 times and about 4-5 times those of commercially-available lidocaine patch preparation comprising lidocaine in the same amount (Lioderm®), respectively.

### EXAMPLES

Hereinafter, the present disclosure is described more specifically with reference to Examples.

### (Example 1)

### [Preparation of patch preparation comprising LC-LA salt and diclofenac sodium and measurement of skin permeation amount]

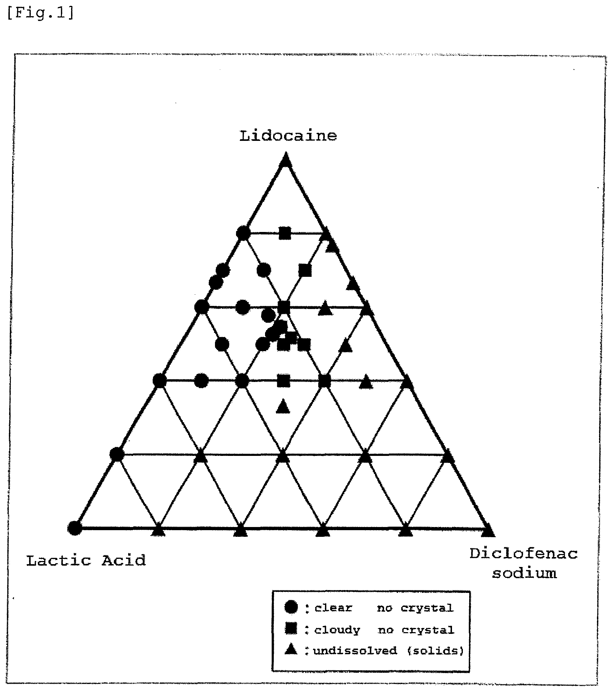

Each ingredient was weighed according to the composition (% by weight) in Tables 1-3 below to prepare patch preparations comprising an equimolar salt of lidocaine-lactic acid and diclofenac sodium. When mixing the ingredients, the mixed ratio of lidocaine, lactic acid and diclofenac sodium, which shows the solution state without the precipitation of crystals of lidocaine and diclofenac sodium, was studied. In the preparation of patch preparations, lidocaine and lactic acid were mixed and then diclofenac sodium was mixed thereto after lactic acid salt of lidocaine which is liquid at ambient temperature was formed. The skin permeation amounts of lidocaine and diclofenac for each of the resulting patch preparations were measured using Franz cell according to a conventional method. The skin on the back of miniature pig was used in the study. The phase diagram for three component mixtures is shown in Fig.1 and the results of the measured skin permeation amounts of the patch preparations are shown in Tables 1 to 3, respectively.

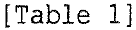

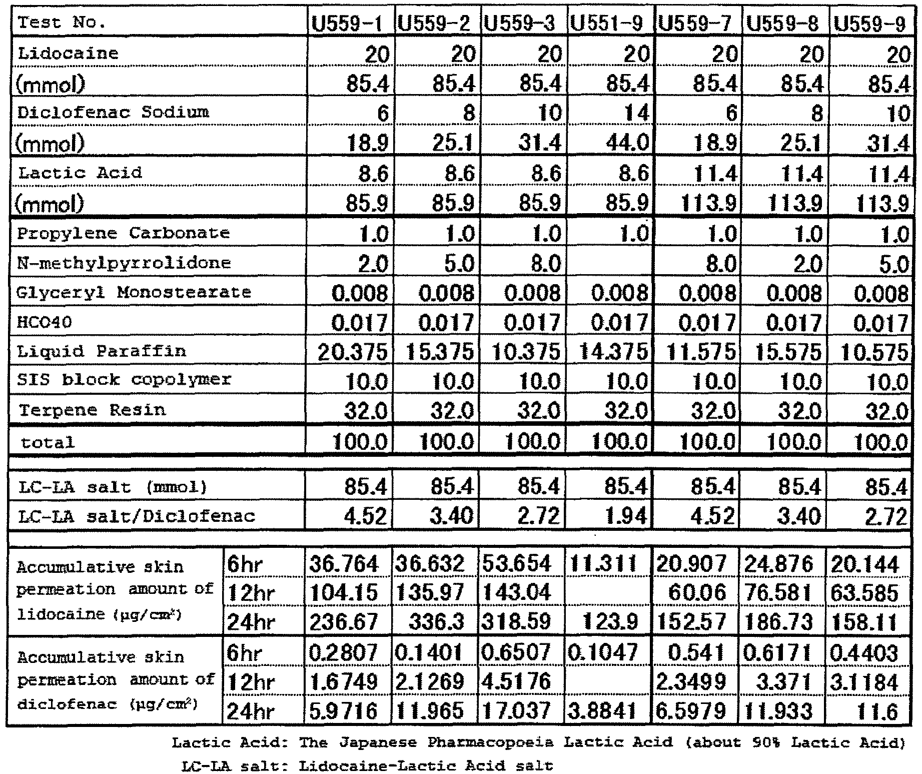

The skin permeation amounts of lidocaine and diclofenac were increased or decreased depending on the value of LC-LA salt/diclofenac, and the maximal value of the skin permeation appeared when the value of LC-LA salt/diclofenac was approximately 3. In particular, Test No. U551-9 showed the worst skin permeability of diclofenac despite the highest concentration of diclofenac sodium. This result is thought to suggest that the amount of LC-LA salt was lower as compared to that of diclofenac.

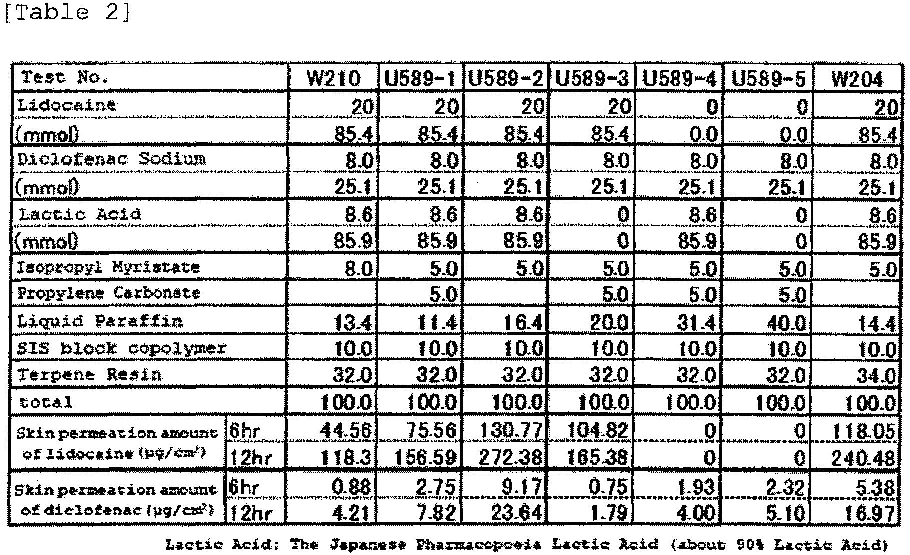

The skin permeation amount of diclofenac in the preparation comprising any of lidocaine or lactic acid was lower than that of the preparation comprising neither lidocaine nor lactic acid. It was suggested that the skin permeability of diclofenac were inhibited by the formation of lidocaine-diclofenac salt. In addition, it is thought that diclofenac is not sufficiently dissolved in lactic acid and the crystallization of diclofenac occurs, and thus the skin permeability of diclofenac is inhibited.

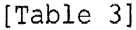

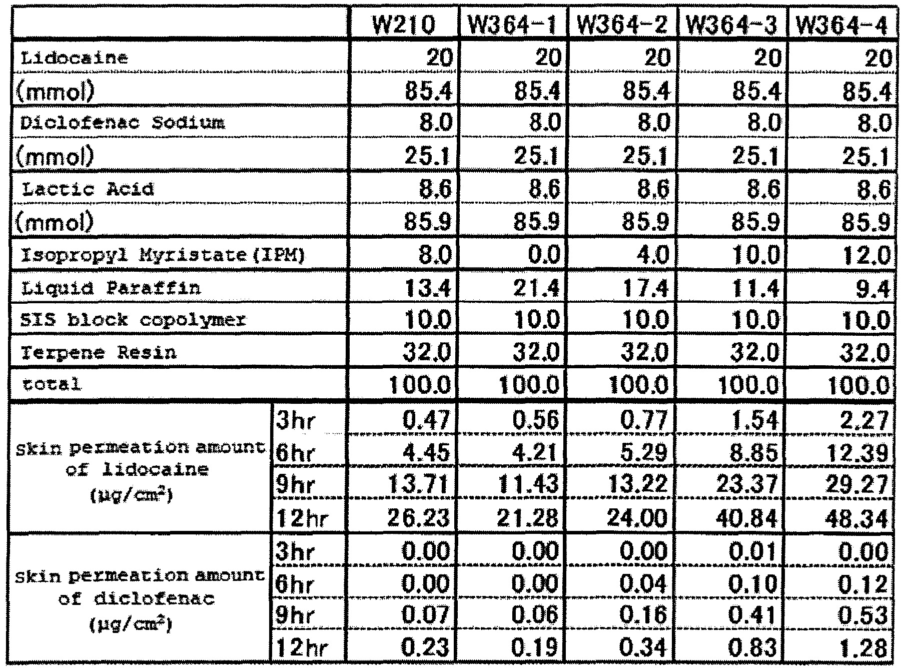

The skin permeation amounts of lidocaine and diclofenac (n=4) were increased depending on the amount of isopropyl myristate (IPM). As a result, it was shown that the transdermal absorbability of lidocaine and diclofenac was further enhanced by the addition of isopropyl myristate.

### (Example 2)

### [Measurement of skin permeation amounts of commercially-available lidocaine patch preparation and diclofenac patch preparation as well as comparison of the skin permeation amounts with those of the above preparations]

According to similar method to that of the above preparation, the skin permeation amounts of commercially-available lidocaine patch preparation (Lidoderm®) and commercially-available diclofenac patch preparation (Flector®) were measured to compare the measured skin permeation amounts with those of the above preparations. The skin permeation amounts of commercially-available Lidoderm® and Flector® were measured for each preparation group of (1) U559-1 to U559-3, U559-8 and U559-9; (2) U551-9; (3) W210; (4) U589-1 to U589-5; (5) W204 and (6) W210 and W364-1 to W364-4. The skin permeation amounts of Lidoderm® and Flector®, and the ratio of the skin permeation amounts of each preparation to those of Lidoderm® and Flector® are shown below. The skin permeation amounts of Lidoderm® and Flector® in the above (1)-(6) are shown in Table 4.

**[Table 4]**

| (1) U559-1 to U559-3, U559-8, U559-9 | | | | | |
|---|---|---|---|---|---|
| | Lidoderm | | | Flector | |
| Skin permeation amount of lidocaine (µg/cm²) | 6hr | 24.27 | Skin permeation amount of diclofenac (µg/cm²) | 6hr | 0.26 |
| | 12hr | 76.18 | | 12hr | 1.87 |
| | 24hr | 192.99 | | 24hr | 6.96 |

| (2) U551-9 | | | | | |
|---|---|---|---|---|---|
| | Lidoderm | | | Flector | |
| Skin permeation amount of lidocaine (µg/cm²) | 6hr | 21.75 | Skin permeation amount of diclofenac (µg/cm²) | 6hr | 0.36 |
| | 12hr | - | | 12hr | - |
| | 24hr | 105.08 | | 24hr | 1.52 |

| (3) W210 | | | | | |
|---|---|---|---|---|---|
| | Lidoderm | | | Flector | |
| Skin permeation amount of lidocaine (µg/cm²) | 6hr | 15.19 | Skin permeation amount of diclofenac (µg/cm²) | 6hr | 0.96 |
| | 12hr | 38.77 | | 12hr | 3.17 |

| (4) U589-1 to U589-5 | | | | | |
|---|---|---|---|---|---|
| | Lidoderm | | | Flector | |
| Skin permeation amount of lidocaine (µg/cm²) | 6hr | 47.2 | Skin permeation amount of diclofenac (µg/cm²) | 6hr | 1.78 |
| | 12hr | 108.11 | | 12hr | 5.88 |

| (5) W204 | | | | | |
|---|---|---|---|---|---|
| | Lidoderm | | | Flector | |
| Skin permeation amount of lidocaine (µg/cm²) | 6hr | 66.41 | Skin permeation amount of diclofenac (µg/cm²) | 6hr | 3.39 |
| | 12hr | 151.86 | | 12hr | 9.09 |

| (6) W210, W364-1 to W364-4 | | | | | |
|---|---|---|---|---|---|
| | Lidoderm | | | Flector | |
| Skin permeation amount of lidocaine (µg/cm²) | 6hr | 24.27 | Skin permeation amount of diclofenac (µg/cm²) | 6hr | 0.26 |
| | 12hr | 76.18 | | 12hr | 1.87 |

| (7) U559-1 to U559-3, U559-8, U559-9 | | | | | |
|---|---|---|---|---|---|
| | Lidoderm | | | Flector | |
| Skin permeation amount of lidocaine (µg/cm²) | 3hr | 0.74 | Skin permeation amount of diclofenac (µg/cm²) | 3hr | 0.00 |
| | 6hr | 5.13 | | 6hr | 0.00 |
| | 9hr | 10.65 | | 9hr | 0.04 |
| | 12hr | 17.43 | | 12hr | 0.08 |

The ratios of the skin permeation amounts of each preparation to those of Lidoderm® and Flector® are shown in Table 5 to Table 7.

**[Table 5]**

| | | U559-1 | U559-2 | U559-3 | U551-9 | U559-7 | U559-6 U559-9 | |
|---|---|---|---|---|---|---|---|---|
| vs Lidoderm | 6hr | 1.51 | 1.51 | 2.21 | 0.52 | 0.86 | 1.02 | 0.83 |
| | 12hr | 1.37 | 1.78 | 1.88 | - | 0.79 | 1.01 | 0.83 |
| | 24hr | 1.23 | 1.74 | 1.65 | 1.18 | 0.79 | 0.97 | 0.82 |
| vs Flector | 6hr | 1.08 | 0.54 | 2.50 | 0.29 | 2.08 | 2.37 | 1.69 |
| | 12hr | 0.90 | 1.14 | 2.42 | - | 1.26 | 1.80 | 1.67 |
| | 24hr | 0.86 | 1.72 | 2.45 | 2.56 | 0.95 | 1.71 | 1.67 |

**[Table 6]**

| | | W210 | U589-1 | U589-2 | U589-3 | U589-4 | U589-5 | W204 |
|---|---|---|---|---|---|---|---|---|
| vs Lidoderm | 6hr | 2.93 | 1.60 | 2.77 | 2.22 | 0.00 | 0.00 | 1.78 |
| | 12hr | 3.05 | 1.45 | 2.52 | 1.53 | 0.00 | 0.00 | 1.58 |
| vs Flector | 6hr | 0.92 | 1.54 | 5.15 | 0.42 | 1.09 | 1.30 | 1.59 |
| | 12hr | 1.33 | 1.33 | 4.02 | 0.30 | 0.68 | 0.87 | 1.87 |

**[Table 7]**

| | | W210 | W364-1 | W364-2 | W364-3 | W364-4 |
|---|---|---|---|---|---|---|
| vs Lidoderm | 3hr | 0.64 | 0.75 | 1.03 | 2.08 | 3.05 |
| | 6hr | 0.87 | 0.82 | 1.03 | 1.99 | 2.41 |
| | 9hr | 1.04 | 1.07 | 1.16 | 1.70 | 2.75 |
| | 12hr | 1.09 | 1.22 | 1.13 | 1.56 | 2.77 |
| vs Flector | 3hr | - | - | - | - | - |
| | 6hr | - | - | - | - | - |
| | 9hr | 1.78 | 1.44 | 3.75 | 9.94 | 12.92 |
| | 12hr | 3.03 | 2.48 | 4.38 | 10.71 | 16.64 |

The above results showed that the preparation of the present disclosure comprising lactic acid salt of lidocaine and diclofenac sodium had more excellent skin permeability as compared to commercially-available Lidoderm® and Flector®.

In Example 1, it was shown that the skin permeation amount of diclofenac in Test No. U551-9 is the worst. However, it is found that Test No. U551-9 sufficiently permeates diclofenac into the skin when comparing with the skin permeation amount of commercially-available Flector®.

### (Example 3)

### [Measurement of plasma lidocaine and diclofenac concentrations from miniature pig]

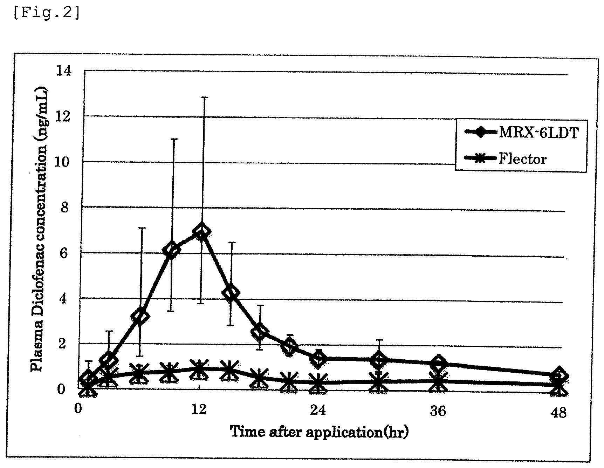

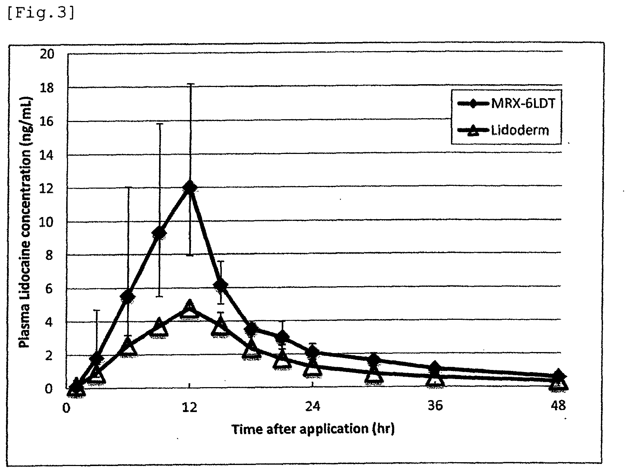

W210 preparation (MRX-6LDT), commercially-available lidocaine patch preparation (Lidoderm®) and commercially-available diclofenac patch preparation (Flector®) were applied to the back of a miniature pig, and then the concentrations of lidocaine and diclofenac in the plasma were measured at each point of the blood collection. In the test, the preparations cut to the 6 cm x 12 cm size were used. The crossover trials of MRX-6LDT group and Lidoderm® + Flector® group were performed. The drug contents and pharmacokinetic parameters of the preparations used in the trials are shown in Table 8. The graphs showing the changes in plasma diclofenac and lidocaine concentrations are shown in Fig. 2 and Fig. 3, respectively.

**[Table 8]**

| | MRX-6LDT | | Flector® | Lidoderm® |
|---|---|---|---|---|
| Active Ingredient | Diclofenac Sodium | Lidocaine | Diclofenac Epolamine | Lidocaine |
| Content (mg) | 82 | 206 | 93 [71.5*] | 360 |
| AUC₀₋₁₂(ng/mL·hr) | 43.38 | 67.87 | 7.61 | 28-56 |
| AUC₀₋ₜ(ng/mL·hr) | 112.18 | 160-09 | 20.05 | 78.24 |
| AUC₀₋ᵢₙₜ(ng/mL·hr) | 125.23 | 168.33 | 31.02 | 83.78 |
| T_{1/2} (hr) | 10.38 | 8.36 | 11.46 | 8.91 |
| Cmax (ng/mL) | 7-48 | 12-00 | 0.96 | 4.76 |
| Tmax (hr) | 11 | 12 | 11 | 12 |

| | | | | |
|---|---|---|---|---|
| * in terms of sodium salt thereof | | | | |

For diclofenac, MRX-6LDT preparation showed about 4 times the AUC_{0-inf} and about 8 times the Cmax of the commercially-available product (Flector®), respectively. For lidocaine, MRX-6LDT preparation showed about 2 times the AUC_{0-inf} and about 2.5 times the Cmax of the commercially-available product (Lidoderm®), respectively.

### (Example 4)

### [Measurement of lidocaine and diclofenac concentrations in skin tissue from miniature pig]

W210 preparation (MRX-6LDT), commercially-available lidocaine patch preparation (Lidoderm®) and commercially-available diclofenac patch preparation (Flector®) were applied to the back of a miniature pig, and then the concentrations of each drug in epidermis, dermis, and subcutaneous tissue were measured. In the test, the preparations cut to the 4 cm x 4 cm size were used. The skin was collected at the time of 3 hours, 6 hours, 12 hours after the application, and 12 hours from the removal of the preparation at 12 hours after the application (24 hours after the application). The results are shown in Table 9.

**[Table 9]**

| Preparation | | W210 | | Flector® | Lidoderm® |
|---|---|---|---|---|---|
| Active Ingredient | | Diclofenac Sodium | Lidocaine | Diclofenac Epolamine | Lidocaine |
| epidermis | 3hr | 30.57 | 112.15 | 11.42 | 89.95 |
| | 6hr | 132.23 | 308.37 | 9.82 | 60.20 |
| | 12hr | 165.32 | 519.76 | 11.8 | 81.62 |
| | 24hr | 55.26 | 153.82 | 7.12 | 18.87 |
| dermis | 3hr | 0.43 | 4.48 | 0.005 | 2.13 |
| | 6hr | 0.79 | 4.63 | 0.003 | 2.65 |
| | 12hr | 0.47 | 7.51 | 0.010 | 2.43 |
| | 24hr | 0.59 | 2.47 | 0.001 | 0.21 |
| subcutaneous tissue | 3hr | 0.12 | 0.78 | 0.038 | 0.47 |
| | 6hr | 0.12 | 0.65 | 0.001 | 0.66 |
| | 12hr | 0.06 | 1.31 | 0.026 | 0.89 |
| | 24hr | 0.06 | 0.38 | 0.003 | 0.07 |

For diclofenac, MRX-6LDT showed about 10 times the tissue concentration of Flector® in epidermis and subcutaneous tissue and about 170 times the tissue concentration of Flector® in dermis. Also, MRX-6LDT showed about 10 times the AUC₀₋ₜ of Flector® in epidermis and subcutaneous tissue and about 90 times the AUC₀₋ₜ of Flector® in dermis.

For lidocaine, MRX-6LDT showed about 2-4 times the tissue concentration of Lidoderm® in epidermis, dermis and subcutaneous tissue. Also, MRX-6LDT showed about 3-5 times the AUC₀₋ₜ of Lidoderm® in epidermis, dermis and subcutaneous tissue.

### Industrial Applicability

The external preparation of the present disclosure can enhance the transdermal absorbability of lidocaine by the inclusion of diclofenac or a salt thereof with the lactic acid salt of lidocaine. Also, the external preparation of the present disclosure can produce excellent transdermal absorbability of both ingredients because the transdermal absorbability of diclofenac or a salt thereof is enhanced without the precipitation of crystal of diclofenac. Hence, the external preparation of the present disclosure is extremely useful in the treatment of various pains.

## Claims

1. An external preparation comprising lactic acid salt of lidocaine and diclofenac or a salt thereof, wherein lactic acid salt of lidocaine is contained in an amount of 2-5 moles per mole of diclofenac or a salt thereof.

2. The external preparation of claim 1, wherein the amount of lactic acid salt of lidocaine is 5-40% by weight.

3. The external preparation of claim 1 or 2, wherein the amount of diclofenac or a salt thereof is 1-20% by weight.

4. The external preparation of any one of claims 1-3, further comprising an ester.

5. The external preparation of claim 4, wherein the ester is diethyl sebacate, methyl laurate, diisopropyl adipate, isopropyl myristate, propylene carbonate, or a mixture thereof.

6. The external preparation of any one of claims 1-5, which is matrix-type patch preparation (tape preparation).

7. The external preparation of claim 6, wherein an adhesive layer therein comprises a polymer with a dispersed solution comprising lactic acid salt of lidocaine and diclofenac or a salt thereof.

8. A process for preparing the external preparation of claim 1, comprising:
mixing lidocaine and lactic acid to provide lactic acid salt of lidocaine which is liquid at ambient temperature; and
dissolving diclofenac or a salt thereof into lactic acid salt of lidocaine.

## Patentansprüche

1. Eine externe Zubereitung, umfassend Milchsäuresalz von Lidocain und Diclofenac oder ein Salz davon, wobei das Milchsäuresalz von Lidocain in einer Menge von 2-5 Mol pro Mol Diclofenac oder einem Salz davon enthalten ist.

2. Die externe Zubereitung nach Anspruch 1, wobei die Menge an Milchsäuresalz von Lidocain 5-40 Gew.-% beträgt.

3. Die externe Zubereitung nach Anspruch 1 oder 2, wobei die Menge an Diclofenac oder einem Salz davon 1-20 Gew.-% beträgt.

4. Die externe Zubereitung nach einem der Ansprüche 1-3, ferner umfassend einen Ester.

5. Die externe Zubereitung nach Anspruch 4, wobei es sich bei dem Ester um Diethylsebacat, Methyllaurat, Diisopropyladipat, Isopropylmyristat, Propylencarbonat oder ein Gemisch davon handelt.

6. Die externe Zubereitung nach einem der Ansprüche 1-5, welche eine matrixartige Pflasterzubereitung (Bandzubereitung) ist.

7. Die externe Zubereitung nach Anspruch 6, wobei eine Klebeschicht darin ein Polymer mit einer dispergierten Lösung umfasst, umfassend Milchsäuresalz von Lidocain und Diclofenac oder ein Salz davon.

8. Ein Verfahren zur Herstellung der externen Zubereitung nach Anspruch 1, umfassend:
Mischen von Lidocain und Milchsäure, um Milchsäuresalz von Lidocain bereitzustellen, das bei Raumtemperatur flüssig ist; und
Auflösen von Diclofenac oder eines Salzes davon in Milchsäuresalz von Lidocain.

## Revendications

1. Préparation externe contenant un sel d'acide lactique de lidocaïne et du diclofénac ou un sel de celui-ci, où le sel d'acide lactique et de lidocaïne est contenu en une quantité de 2-5 moles par mole de diclofénac ou d'un sel de celui-ci.

2. Préparation externe selon la revendication 1, où la quantité de sel d'acide lactique de lidocaïne est de 5-40 % en poids.

3. Préparation externe selon la revendication 1 ou 2, où la quantité de diclofénac ou d'un sel de celui-ci est de 1-20 % en poids.

4. Préparation externe selon l'une quelconque des revendications 1-3, comprenant en outre un ester.

5. Préparation externe selon la revendication 4, où l'ester est le sébaçate de diéthyle, le laurate de méthyle, l'adipate de diisopropyle, le myristate d'isopropyle, le carbonate de propylène ou un mélange de ceux-ci.

6. Préparation externe selon l'une quelconque des revendications 1-5, qui est une préparation de timbre de type à matrice (préparation de type ruban).

7. Préparation externe selon la revendication 6, où une couche adhésive dans celle-ci comprend un polymère avec une solution dispersée comprenant un sel d'acide lactique de lidocaïne et du diclofénac ou un sel de celui-ci.

8. Procédé pour préparer la préparation externe selon la revendication 1, comprenant:
le mélange de lidocaïne et d'acide lactique pour fournir un sel d'acide lactique de lidocaïne qui est liquide à la température ambiante;
et
la dissolution de diclofénac ou d'un sel de celui-ci dans le sel d'acide lactique de lidocaïne.
